# EUROPEAN PATENT SPECIFICATION

(11) **EP 3 982 899 B1**
(45) Date of publication and mention of the grant of the patent: **28.01.2026**
(21) Application number: 19932429.4
(22) Date of filing: 11.06.2019
(51) Int. Cl.: A61F 13/495, A61F 13/533, A61F 13/15

(54) **ABSORBENT ARTICLE AND METHOD FOR MANUFACTURING ABSORBENT ARTICLE**
SAUGFÄHIGER ARTIKEL UND VERFAHREN ZUR HERSTELLUNG DES SAUGFÄHIGEN ARTIKELS
ARTICLE ABSORBANT ET PROCÉDÉ DE FABRICATION DE L'ARTICLE ABSORBANT

(43) Date of publication of application: 20.04.2022
(73) Proprietor: Essity Hygiene and Health Aktiebolag, 405 03 Göteborg (SE)
(72) Inventor: VARTIAINEN, Kent, 443 92 LERUM (SE); BADERSTEDT, Julia, 405 03 GÖTEBORG (SE); ASAYESH, Yasha, 405 03 GÖTEBORG (SE); CORNELIUSSON, Helena, 405 03 GÖTEBORG (SE)
(74) Representative: Hoffmann Eitle
(86) International application number: PCT/SE2019/050550
(87) International publication number: WO 2020/251434

(56) References cited:
- US-A- 5 558 660
- US-A1- 2002 111 594
- US-A1- 2005 107 761
- US-A1- 2017 000 658
- US-A1- 2018 168 888
- US-A1- 2018 289 562

## Description

### TECHNICAL FIELD

The disclosure relates to an absorbent article comprising a liquid-permeable topsheet, a liquid impermeable backsheet and an absorbent core positioned intermediate the liquid permeable topsheet and the liquid impermeable backsheet.

### BACKGROUND

Wearable and disposable absorbent articles, for example in the form of diapers, incontinence articles and feminine hygiene pads offer the benefit of receiving and containing urine, feces and/or other bodily exudates (e.g. menses, mixture of feces and urine, mixture of menses and urine, etc.) are well known in the art. Such articles are used to absorb, distribute and store various types of body exudates while providing a high level of comfort and sense of dryness to the wearer during use.

A conventional disposable absorbent article in the form of a diaper is normally designed with an absorbent core which is sandwiched between a topsheet and a backsheet. The article is arranged along a longitudinal axis and along a transversal axis which extends in a perpendicular direction in relation to the longitudinal axis. Furthermore, the article can be divided into a front portion, a back portion and a crotch portion.

When a user of absorbent article exudates fecal material (e.g. runny bowel movement, a mixture of bowel movement and urine, etc.) it is important that the absorbent article can handle the bodily exudates i.e. absorb and retain the fecal material within the absorbent article. Absorbent articles can efficiently handle this by having proper fit around the waist and the legs of the user, by having proper absorption of the bodily exudates and/or by having means for preventing fecal materials to leak out of the absorbent article.

Means for preventing fecal materials to leak out are frequently used in the art. One method is to prevent leakage of fecal material by providing a pocket on the back part of the absorbent article for collecting fecal material and avoid leakage through the waist of the user. The drawback with pockets and other means for prohibiting leakage of fecal materials is that during a rapid flow of bodily exudates the means for preventing leaks may not efficiently prohibit the fecal materials to be gathered and retained within the absorbent article. This problem occurs often when the flow of fecal material is high and/or when the absorbent core is expanded. A higher flow of fecal material often occurs when a person suffers from a virus or bacterial infection, allergic reaction or when performing a medicine cure, drinking breastmilk or a breastmilk substitute etc. The absorbent core may be expanded during the exudate of the fecal material if the user of an absorbent article for example has urinated prior to exudation of the fecal material.

Accordingly, there is an interest to provide means for retaining fecal materials within the absorbent article more efficiently i.e. means that prohibit the fecal materials to leak out of the waist of the diaper despite of the flow of the fecal material and/or despite the state of the absorbent core of the absorbent article.

A previously known absorbent article of the above-mentioned kind is disclosed in the patent document EP2750648A1. This document discloses an incontinence article of pants-type for holding bodily excretions. The rear end area of the absorption structure is covered by a flat-material section which is joined inseparably to the crotch portion to form a pocket.

Even though the article disclosed in EP2750648A1 fulfills certain requirements related to preventing fecal materials to leak, there is still a need for further improvements in order to provide an absorbent article being able to provide this feature in a more efficient manner.

US 2005/107761 A1 discloses features falling under the preamble of claim 1. US 2002/111594 A1, US 2018/168888 A1, and US 2017/000658 A1 are further prior art.

### SUMMARY

The present disclosure is based on the insight that the ability of an absorbent article such as for example a diaper to avoid leakage may be challenged under certain conditions.

In accordance with the invention there is provided an absorbent article according to claim 1, and a method according to claim 14. The dependent claims specify preferred but optional features.

The present invention provides an absorbent article comprising a liquid-permeable topsheet, a liquid impermeable backsheet and an absorbent core positioned intermediate the liquid permeable topsheet and the liquid impermeable backsheet, each layer having a skin-facing side and a garment-facing side, wherein said absorbent core comprises a thickness, said absorbent article being arranged along a longitudinal axis and a transversal axis extending in a perpendicular direction in relation to the longitudinal axis, and said absorbent article defining a front portion having a front edge, a back portion having a back edge and a crotch portion intermediate said front portion and said back portion, wherein said back portion of said absorbent article further comprises a transversally extending capture layer, wherein said capture layer comprises an outboard transversal edge facing said back edge and two longitudinal edges being sealed to any part of said absorbent article, wherein said capture layer further comprises an inboard transversal edge facing said front edge, said inboard transversal edge being unsealed for gathering of feces, wherein said capture layer is positioned to at least partially cover the skin-facing side of the topsheet, wherein said absorbent core comprises at least one thickness, wherein at least said back portion of said absorbent article further comprises at least one centrally oriented zone in said absorbent core, said zone comprising a thickness, wherein said zone is at least partially recessed, resulting in said zone having a generally lower thickness in relation to the thickness of surrounding parts of the absorbent core.

The at least one zone extends longitudinally from the back transversal edge of the absorbent core.

Further, said at least one zone may have a lower basis weight in relation to surrounding parts of the absorbent core.

The inboard transversal edge of the capture layer at least partially overlaps the at least one zone.

The at least one zone may further comprise a length, wherein the absorbent core comprises a length, wherein the length-ratio between the at least one zone and the absorbent core may be within the interval 0.04-1.

The at least one zone may further comprise a width and the absorbent core may comprise a width, wherein the width-ratio between the at least one zone and the absorbent core may be within the interval 0.02-0.8, preferably within the interval 0.02-0.28.

The capture layer may comprise at least one transversally extending elastic strand positioned between a pair of sealed layers of material.

Furthermore, the at least one zone may extend along the entire longitudinal length of the absorbent core.

The at least one zone may be fully recessed.

The at least one zone may be shaped generally as a semi-circle, a U-shape, a V-shape, a circular shape, an oval shape or a rectangular shape extending towards the front edge in the longitudinal direction.

The absorbent core may be enclosed by a core cover comprising a top-layer and a bottom-layer, wherein at least a part of the topsheet or an intermediate layer is joined to said top-layer, wherein the top-layer and the bottom-layer are joined together in at least the area comprising the fully recessed zone.

The basis weight of the at least one zone may be within the interval of 0-200 g/m ², wherein the basis weight of surrounding parts of the absorbent core may be within the interval of 50-800g/m ².

The said at least one zone may be divided in said back portion into two oblong channels extending longitudinally to at least said crotch portion of said absorbent article.

The absorbent article may comprise two centrally oriented zones extending from said back portion to at least said crotch portion.

There is further also provided a method for manufacturing an absorbent article, comprising a liquid-permeable topsheet, a liquid impermeable backsheet and an absorbent core positioned intermediate the liquid permeable topsheet and the liquid impermeable backsheet, each layer having a skin-facing side and a garment-facing side, said absorbent article being arranged along a longitudinal axis and a transversal axis extending in a perpendicular direction in relation to the longitudinal axis, and said absorbent article defining a front portion having a front edge, a back portion having a back edge and a crotch portion intermediate said front portion and said back portion, said method comprising:
- forming a capture layer on said back portion of said absorbent article, wherein said capture layer comprises an outboard transversal edge facing said back edge and two longitudinal edges being sealed to any part of said absorbent article, wherein said capture layer further comprises an inboard transversal edge facing said front edge, said inboard transversal edge being unsealed for gathering of feces, wherein said capture layer is positioned to at least partially cover the skin-facing side of the topsheet
- forming an absorbent core comprising at least one thickness, wherein wherein at least said back portion of said absorbent core further comprises at least one centrally oriented zone, said zone being at least partially recessed, resulting in said zone having a lower thickness in relation to surrounding parts of the absorbent core.

Further advantages and advantageous features of the disclosure are disclosed in the following description.

### BRIEF DESCRIPTION OF THE DRAWINGS

The disclosure will be described in greater detail below with reference to the figures shown in the appended drawings.
- Figure 1A: Shows a top view of an embodiment of an absorbent article.
- Figure 1B: Shows a cross-sectional front view of the capture layer and the absorbent core of the absorbent article in Figure 1A.
- Figure 1C: Shows a cross-sectional front view of the capture layer, the topsheet, cover and the absorbent core of the absorbent article in Figure 1A.
- Figure 2A: Shows a top view of an embodiment of an absorbent article.
- Figure 2B: Shows a cross-sectional front view of the capture layer and the absorbent core of the absorbent article in Figure 2A.
- Figure 3: Shows a top view of an embodiment of an absorbent article.
- Figure 4: Shows a top view of an embodiment of an absorbent article.
- Figure 5: Shows a top view of an embodiment of an absorbent article.
- Figure 6: Shows a top view of an embodiment of an absorbent article.

### DETAILED DESCRIPTION

Different aspects of the present disclosure will be described more fully hereinafter with reference to the enclosed drawings. The embodiments disclosed herein can, however, be realized in many different forms and should not be construed as being limited to the aspects set forth herein.

The term "absorbent article" refers to a product that is configured to be worn on or around the lower torso of the body of a human wearer. The absorbent article is intended to absorb and/or contain body exudates, such as urine, feces and menses fluid. Absorbent articles include for example open absorbent articles, pant-type absorbent articles and belt-type absorbent articles, being able to be worn without the support from underwear. The absorbent article may be a disposable absorbent article.

The "body facing side" of the absorbent article refers to the side facing the wearer during use and the "garment-facing side" refers to the side facing away from the wearer during use. The garment facing side and the body facing side are thus facing away from one another.

An "open type absorbent article" relates to an absorbent article having fastening elements arranged to connect the front portion with the back portion when the article is being worn.

A "pant type absorbent article" relates to an absorbent article wherein the front portion and back portion are permanently connected prior to use, forming a waist opening and a pair of leg openings.

The term "capture layer" refers to at least one sheet of suitable material arranged on the skin-facing side of the topsheet to form a pocket-like structure. The opening of the pocket is arranged to gather body exudates from a user.

The term "zone" refers to a part of the absorbent core that differs in properties and/or dimensions in relation to surrounding parts of the absorbent core not encompassing the zone.

The term "topsheet" refers to a fluid pervious body facing structure that may be sufficiently liquid-permeable to allow discharged body fluids to penetrate through its thickness. The topsheet may be suitably manufactured from a material which is compliant and soft-feeling to the skin of the wearer. The topsheet may consist of a single layer or have a laminate structure comprising a plurality of layers, for example, two or more layers. The layers may be made of the same material, or some or all the layers may be made of different materials. The topsheet material may be of a nonwoven, a film or a laminate thereof or of a foam. The material may be of thermoplastic synthetic or natural fibers, or a mixture thereof. Natural fibers may be e.g. cotton or pulp fibers and synthetic fibers may be e.g. of a polyester such as PET or a polyolefin such as polyethylene or polypropylene, or a combination of these fibers. The nonwoven may be formed by a variety of different processes, such as spun bonding, air laying, melt blowing or bonded carded web formation processes. The nonwoven layer may be made of an SMS (spun bond/melt blown/spun bond) or SS (spun bond/ spun bond) nonwoven material of polypropylene or bicomponent fibers of polypropylene and polyethylene, or of a combination of such materials. The topsheet layer or layers may be apertured or non-apertured and may have imparted hydrophilic properties to improve liquid drainage of the structure. The topsheet may have a basis weight in the range of 5-50 g/m ².

The term "backsheet" refers to the sheet which is facing away from the user during use and is opposite to the body facing topsheet layer of the absorbent article. The backsheet may be a liquid-impermeable or fluid repellant structure. The backsheet material may be of a breathable or non-breathable material such as a of a film, a nonwoven or a laminate thereof, or a foam. The backsheet may have a laminate structure comprising a liquid barrier film and a nonwoven layer arranged on top of each other, wherein the nonwoven layer is arranged at an outer side away from the wearer of the absorbent article when worn. The backsheet may be elastic in any direction.

The film may be of a thermoplastic polymer, e.g. a polyester such as PET or of polyolefins such as of polyethylene or polypropylene, or a mixture thereof. It may be a single layer film or a multi-layer film.

The nonwoven may be of thermoplastic polymer material fibers or filaments e.g. a polyester such as PET or of polyolefins such as of polyethylene or polypropylene, or a mixture thereof. The nonwoven may be formed by a variety of different processes, such as spun bonding, air laying, melt blowing or bonded carded web formation processes. The nonwoven layer may be made of an SMS (spun bond/melt blown/spun bond) or SS (spun bond/ spun bond) nonwoven material of polypropylene or bicomponent fibers of polypropylene and polyethylene, or of a combination of such materials.

Forming the liquid barrier film of a plastic material, such as a thermoplastic film material, allows for a particularly good printability of the liquid barrier sheet. The liquid barrier sheet may also contain cellulose fibers.

The liquid barrier film may consist of a single layer or have a laminate structure with a plurality of layers, e.g., two or more layers, three or more layers, or four or more layers. The layers of the laminate structure may be laminated, bonded or attached to each other, for example, by thermo and/or mechanical bonding, such as thermo-sealing, ultrasonic bonding, such as ultrasonic welding, an adhesive or adhesives, stitching or the like.

The liquid barrier sheet may be a breathable microporous film. The microporous film may be made of a material comprising at least two basic components, namely a thermoplastic elastomeric polyolefin polymer and a filler. These components and, in some embodiments, additional other components may be mixed together, heated and subsequently extruded into a mono-layer or multi-layer film using any one of various film-producing processes, such as cast embossed, chill and flat cast, and blown film processes.

The term "absorbent core" refers to the unit placed intermediate the topsheet and the backsheet. The absorbent core can be of any conventional kind. Examples of common absorbent materials used in absorbent cores are cellulosic fluff pulp, tissue layers, highly absorbent polymers (so called superabsorbents), absorbent foam materials, absorbent nonwoven materials or the like. It is common to combine cellulosic fluff pulp with superabsorbent polymers (SAP) in an absorbent core.

It is conventional in absorbent articles to have an absorbent core comprising layers of different properties with respect to liquid receiving capacity, liquid distribution capacity and storage capacity. The thin absorbent bodies, which are common in for example baby diapers and incontinence articles, often comprise a compressed mixed or layered structure of cellulosic fluff pulp and superabsorbent polymers. The size and absorbent capacity of the absorbent core may be varied to be suited for different absorbent article sizes or different uses such as for infants or for adult incontinent persons. The term "absorbent core" as discussed herein, does not encompass the core cover. The form of the absorbent core may be varied, as will be discussed herein.

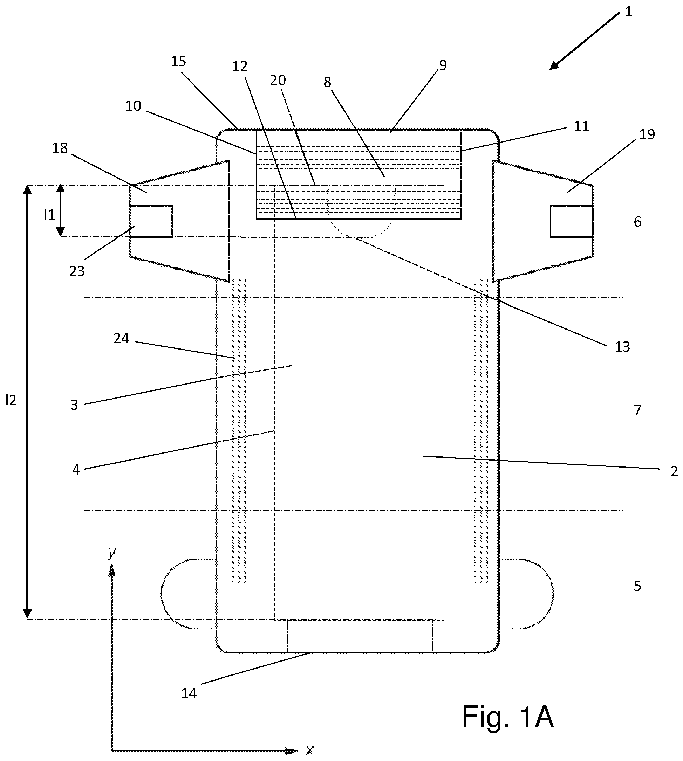

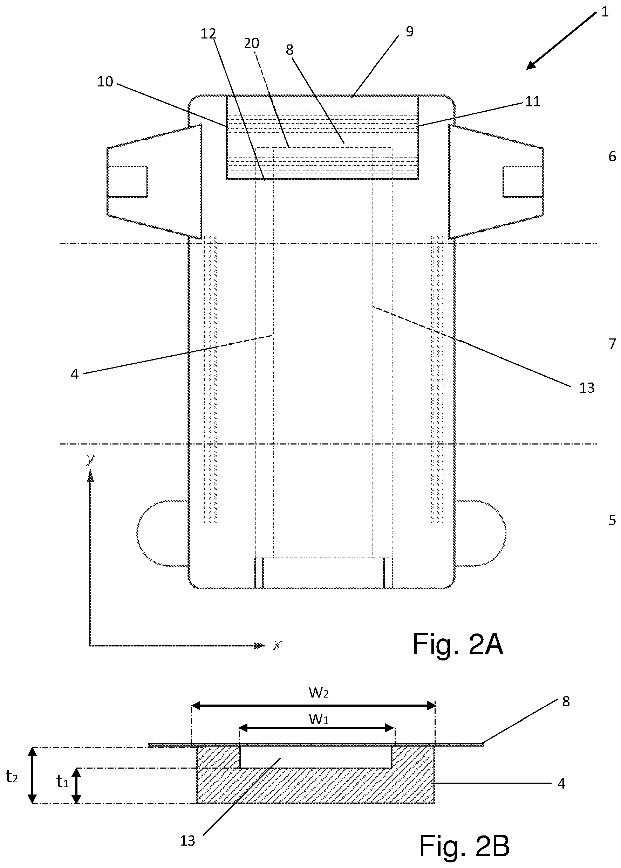

With initial reference to Figure 1A and Figure 2, there is shown a view from above of an absorbent article 1 in the form of a baby diaper. The absorbent article is shown in Figure 1A and Figure 2 in a flat and unfolded state. Also, the absorbent article 1 is based on an absorbent structure for absorbing body exudates from a wearer to provide a dry and comfortable fit for the wearer.

As shown in Figure 1A, the absorbent article 1 comprises a liquid permeable topsheet 2, a liquid impermeable backsheet 3 and an absorbent core 4 positioned intermediate the liquid permeable topsheet 2 and the liquid impermeable backsheet 3. The absorbent article 1 is arranged along a longitudinal axis y and a transversal axis x extending in a perpendicular direction in relation to the longitudinal axis y, in other words, the absorbent article 1 has a length extension in the longitudinal direction y and a width extension in the transverse direction x. The topsheet 2 is arranged at the surface of the article 1, i.e. at the side which is facing the wearer, whereas the backsheet 3 is arranged at the underside of the article 1. The absorbent article 1 further comprises an absorbent core 4 positioned intermediate the liquid permeable topsheet 2 and the liquid impermeable backsheet 3. Furthermore, both the topsheet 2 and the backsheet 3 extend laterally outside of the absorbent core 4 along the entire perimeter of the article 1 as disclosed in figures 1A and 2.

Further, an acquisition and distribution layer (ADL) may be arranged as an intermediate layer between the absorbent core 4 and the topsheet 2. The acquisition layer may be adapted to quickly receive and temporarily store discharged liquid before it is absorbed by the absorbent core 4. The polymer materials of the ADL may be non-absorbent materials which do not retain any fluid in the material itself. The function of the ADL is to provide the absorbent article 1 with temporary fluid holding capacity and to distribute fluid in the article. As the polymer materials in the fluid flow control structure may be hydrophobic and may have a wetting angle (θ) of 90° or close to 90° implying that they have no or very low wettability when contacted with aqueous fluids. The components of the fluid flow control structure may be treated to lower the wetting angle and render them hydrophilic, i.e. wettable by body fluids. A perfectly wettable material has a wetting angle (θ) of 0°. Any commonly known method for rendering a hydrophobic material hydrophilic may be used, such as treatment with surfactants, plasma or corona treatment, etc. A hydrophilic material facilitates liquid penetration and draining, thus maintaining free volume capacity for the next gush of liquid. An ADL may be composed of for example air laid, nonwoven, high loft nonwoven or foam materials. An air laid may be produced with fluff, wood pulp, and here the fluff fibres are dispersed into a fast-moving air stream and condensed onto a moving screen by means of pressure and vacuum. The ADL may be of a non-perforated nonwoven material or a perforated material.

As disclosed in Figure 1A the absorbent article 1 may be an open-type diaper. However, the invention may be subject to any type of absorbent article 1 such as a pant-type absorbent article 1. The absorbent article 1 of the present invention may be subject to baby diapers, but also incontinence diapers for elderly. Figure 1A further discloses that the absorbent article 1 has three portions, a front portion 5, a back portion 6 and a crotch portion 7 intermediate the front portion 5 and the back portion 6. The front portion 5 defines the part of the absorbent article 1 that is situated on the front side of the user and the back portion 6 defines the part of the absorbent article 1 that is situated on the back side of the user, in other words, the front portion 5 at least partially covers the abdomen of the user and the back portion 6 at least partially covers the rear of the user. It is further disclosed in Figure 1A that the front portion 5 of the absorbent article 1 defines a front edge 14 and the back portion of the absorbent article defines a back edge 15. The front edge 14 and the back edge 15 are defined as the edges of the respective short sides of the absorbent article 1, front edge 14 being on the short side of the front portion 5 and the back edge 15 being on the short side of the back portion 6.

Furthermore, the absorbent article 1 may have a pair of standing gathers, which extend upwardly generally about respective side edges of the absorbent core 4 at the side of the topsheet 2. Such a standing gather defines a barrier or wall at the respective side edges of a central crotch portion 7 of the absorbent article 1, which acts to prevent or retard lateral flow of body fluidic material such as urine or fluidic fecal material.

The standing gathers may run substantially parallel to the longitudinal edges at the crotch portion 7 in a longitudinal direction of the absorbent article 1. The standing gathers may contain elastics and may include an elastic material, such as an elastic thread, yarn or ribbon material, and may be elastically gathered at least in their center portions, which provide means for a good fit so as to prevent any leakage. The standing gathers may extend in the longitudinal direction y over the entire length of the absorbent article 1. It will be appreciated, however, that, the standing gathers may be shorter.

Furthermore, at least one longitudinal leg elastic element 24 may be arranged in the crotch portion 7 at each longitudinal side that may extend at a side outside each one of the standing gathers along the longitudinal direction y thereof. Each longitudinal side may at least in part be made of the standing gather sheet material. Upon use of the absorbent article 1, the elastic elements may provide that the longitudinal sides are elastically gathered and serve as leg elastic, which in turn provides an extra leakage barrier and a good fit to the wearer.

Figure 1A further discloses a transversally extending capture layer 8 extending on the back portion 6 of the absorbent article 1. The term "transversally extending" indicates that the capture layer 8 extends in the direction of the transversal axis x. Further, as shown in Figure 1A the capture layer 8 may traverse the absorbent core 4 in the direction of the transversal axis x, in other words the capture layer 8 may have a rectangular shape wherein the long sides of the rectangle traverses the absorbent core 4, however the capture layer 8 may have other shapes such as a squared shape, triangular shape, oval shape, u-shape or any other shape that is suitable for the purpose of the invention. Further, the capture layer 8 comprises an outboard transversal edge 9 and two longitudinal edges 10, 11, where the outboard transversal edge 9 faces the back edge 15 of the absorbent article as shown in Figure 1A. However, the capture layer 8 may also be constructed such that there is a space between the outboard transversal edge 9 of the capture layer 8 and the back edge 15 of the absorbent article 1. The transversal edges of the capture layer are the edges that traverse the absorbent core 4 in the direction of the transversal axis x, as shown in Figure 1A. Figure 1A further shows that the capture layer 8 may comprise two longitudinal edges 10, 11 that are parallel with the longitudinal axis y. As disclosed in Figure 1A and Figure 2A the capture layer 8 may also traverse the back transversal edge 20 of the absorbent core 4 in the direction of the longitudinal axis y, the back transversal edge 20 of the absorbent core 4 is intermediate the outboard transversal edge 9 of the capture layer 8 and the inboard transversal edge 12 of the capture layer 8.

The outboard transversal edge 9 and the two longitudinal edges 10, 11 of the capture layer are sealed to any part of the absorbent article 1. It may be sealed by bonding technique such as glue, mechanical bonds, thermal bonds or any other suitable sealing method. Based on this, the outboard transversal edge 9 and the two longitudinal edges 10, 11 may be sealed to any part of the back portion 5 of the absorbent article 1 such as to the topsheet 2 or the standing gathers or a combination thereof. The inboard transversal edge 12 is not sealed to any part of the absorbent article 1 or may be partially sealed to any part of the absorbent article 1. Based on this, the capture layer 8 is constructed to function as a pocket, having three sealed side edges, and one unsealed edge. It should be noted that the inner parts of the capture layer 8 has to be at least partially unsealed in order for the pocket-function to operate i.e. all edges except the inboard transversal edge 12 are sealed and the other portions of the capture layer 8 form a cavity/pocket for collection of body exudates. The advantage of this construction is that the capture layer 8 is able to gather bodily exudates from a user flowing towards the back edge 15 of the absorbent article 1, thereby preventing unwanted leakage. As disclosed in Figure 1A the capture layer 8 is situated on the skin-facing side of the topsheet 2.

The capture layer 8 may comprise of at least one transversally extending elastic strand positioned intermediate at least one pair of sealed layers of sheet material, this structure may be achieved when the elastic layer is stretched in at least one direction before laminating it together with two or more inelastic layers. After the tension is removed from the elastic layer it can freely retract to its un-tensioned state. The capture layer 8 may consist of a first sheet and a second sheet and at least one elastic strand of an elastomeric construction with adhesive composition adhered between the first facing sheet and the second facing sheet.

Further, the pair of sealed layers of sheet material could be two or more sheets placed upon one another or consist of a folded structure. Furthermore, if the absorbent article 1 is in the form of a pant diaper the capture layer 8 may be a part of the waist section of the absorbent article 1, in other words, the front region 5 and back region 6 of the pant-diaper being permanently connected prior to use may also form a pocket on the back portion 5 of the absorbent article 1. The capture layer 8 may be corrugated. The capture layer 8 comprises a garment facing side, and a skin-facing side. The material of the capture layer may be a nonwoven material, a film, a laminate, an elastic film, an elastic laminate or a combination thereof. An example of a material combination for the structure of the capture layer 8 is that the garment facing side of the capture layer 8 consists of a film and the skin-facing side of the capture layer 8 consists of a nonwoven material. The benefit of such a material formation is that the film may be more liquid-impermeable and hence help the body exudates to stay within the pocket. Further, the benefit of having a nonwoven layer on the skin-facing side of the capture layer 8 is more comfortable against the skin of the user of the absorbent article 1.

The inboard transversal edge 12 of the capture layer 8 may have different forms, such as a transversal line as shown in Figure 1A and Figure 2A or it may have a V-shape or a U-shape or a zigzag-shape or any other shape that may be suitable for the inboard longitudinal 12 edge of the capture layer 8.

As seen in Figure 1A the absorbent article 1 may comprise a pair of elastic side panels 18, 19 attached to opposing longitudinal edges of the back portion 5 of the absorbent article 1. The elastic side panels 18, 19 may have any suitable shape, such as rectangular, square, triangular, or trapezoid. The elastic side panels 18, 19 on each side of the article may be of the same size and shape as each other and include the same materials as each other. Each of the elastic side panels 18, 19 includes at least one fastening means 23, e.g. preferably one fastening means 23 as shown in the Figures 1A and 2. The fastening means 23 are substantially inelastic. The fastening means 23 are adapted to fasten to the front portion 5 of the diaper, so that the article will assume a pant-like shape. The fastening means 23 illustrated in Figure 1A are attached to each distal end of each elastic side panel 18, 19 and are intended to be fastened to the garment-facing side of the front portion 5. The fastening means 23 may include an adhesive tape or a mechanical fastener, especially a hook fastener of a hook-and-loop fastening means. A "hook-and-loop fastener" refers to complementary fastening means 23 having a "hook" portion and a "loop" portion and which are refastenable. The term "hook" as used herein refers to any element capable of engaging another element, the so called "loop" portion. The term "hook" is not limited to only "hooks" in its normal sense, but rather encompasses any form of engaging elements, whether unidirectional or bidirectional. The term "loop" is likewise not limited to "loops" in its normal sense, but also encompasses any structure capable of engaging with a "hook" fastener. Examples of "loop" materials are fibrous structures, like nonwoven materials. Hook-and-loop fasteners are for example available from Velcro, USA. Further examples of mechanical fasteners are button and holes or button loops, snap fasteners and the like. Combinations of adhesive and mechanical fasteners may also be provided.

Further, the elastic side panels 18, 19 may be an extension of the capture layer 8 where the capture layer 8 is one sheet with fastening means 23 on its both distal ends located at the longitudinal edges of the capture layer 8, in other words, the elastic side panels 18, 19 and the capture layer 8 may be one interconnected elastic sheet structure that is extending transversally over the width of the absorbent article 1 and further extending transversally outside said width to form the pair of elastic side panels 18, 19. The benefit of having the capture layer 8 and the pair of elastic side panels 18, 19 on one sheet could be that the production and assembly costs of the diaper will be lower since the diaper will require less material and processes during manufacturing.

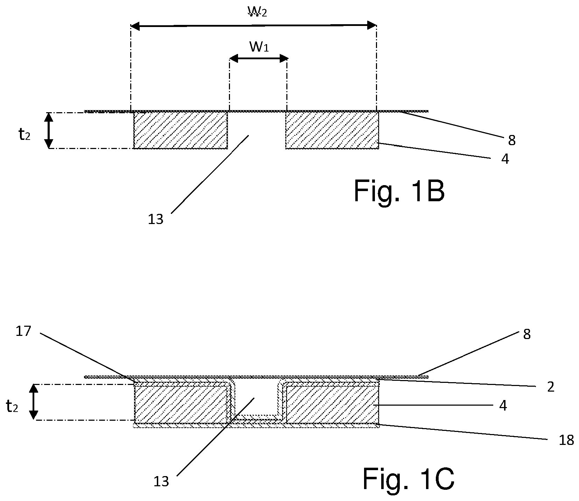

As shown in Figure 2B, Figure 1B and Figure 1C, the absorbent core 4 comprises a thickness t2, and further the absorbent core 4 comprises one centrally oriented zone 13 having a thickness t1, said zone 13 may be at least partially recessed which results in said zone 13 having a lower thickness t1 in relation to the thickness of surrounding parts of the absorbent core t2. The term "surrounding parts of the absorbent core" refers to all the areas of the absorbent core 4 that are not encompassed by the zone 13, in other words, the term refers to the areas of the absorbent core 4 outside of the zone 13. The zone 13 may be recessed as shown in Figure 1B or Figure 2B where there is disclosed cross-sectional front views of the absorbent core 4 and the capture layer 8 wherein the absorbent article 1 of Figure 2B is partially recessed in the centrally oriented zone 13 and the absorbent article of Figure 1B is fully recessed in the centrally oriented zone, hence t1 is not disclosed in the Figure 1B. The zone 13 may extend longitudinally from the back transversal edge 20 of the absorbent core 4 as shown in Figure 1A and Figure 2A. The zone may have a lower basis weight in relation to surrounding parts of the absorbent core 4. In Figure 1A, the zone 13 is fully recessed and has no basis weight i.e. the basis weight of the zone is 0 gsm in Figure 1A, and the basis weight of surrounding parts of the core is > 0 gsm, further Figure 1B and Figure 1C shows that the zone 13 has a thickness t1 = 0 wherein the surrounding parts of the absorbent core 4 has a thickness t2 > 0. This is further disclosed in Figure 1B, where there is shown that the zone 13 is fully recessed and consequently has no thickness i.e. there is only disclosed one thickness t2 in the figure. Figure 1B further shows that the capture layer 8 traverses the zone 13 in the transversal direction.

The benefit of having a lower basis weight in the zone 13 compared to surrounding parts of the core 4 is that the zone 13 may achieve a generally lower thickness t1 compared to the thickness t2 in surrounding parts of the absorbent core 4 by having a lower basis weight. Figure 1B and Figure 2B disclose that the thickness is generally t2 > t1. The basis weight of the zone 13 may be within the interval of 0-200 g/m ², wherein the basis weight of surrounding parts of the absorbent core 4 may be within the interval of 50-800 g/m ².

The thickness may be measured under a pressure of 0.5 kPa, using a thickness gauge foot with dimensions 50 x 50 mm. The thickness is measured on several spots to obtain a representative mean.

The benefit of having a centrally oriented zone 13 with a generally lower thickness t1 compared to the thickness t2 of surrounding parts of the absorbent core 4 is that firstly, the gluteal cleft is centrally located on the body, hence the central location of the zone 13, further the generally lower thickness t1 of the zone 13 results in the pocket formed under the capture layer 8 having more volume to more efficiently gather bodily exudates. Furthermore, when a user wears the absorbent article 1 and subsequently puts pressure on the absorbent core 4 by means of sitting, lying down or any other way, the at least partially recessed zone 13 will contribute to being exposed to a lower pressure N/m ² compared to the surrounding parts of the absorbent article 1, in other words, the force per m ² will be lower in the area of the zone 13 compared to the surrounding parts of the absorbent article 1. This may result in that the surrounding parts of the absorbent article 1 will have a pressure receiving function, as a consequence, the bodily exudates will be prevented to flow in the surrounding parts of the absorbent core 4 and instead flow into the zone 13 and further into the pocket i.e. beneath the capture layer. Hence, the zone acts as a pressure-relieving area. Since the zone area 13 is exposed to a lower pressure and situated centrally, the bodily exudates will tend to flow within the zone area 13 and further into the pocket. Furthermore, generally when a user uses an absorbent article 1 and for instance is sitting down, the least amount of pressure on the absorbent article 1 from the user stems from the groove of the rear of the user, hence when a user exudates fecal material, it tends to flow within the groove of the rear of the user. Based on this, having the zone 13 at least partially aligned with said groove, will create a distance below the garment-facing side of the capture layer 8 and further help fecal material to more efficiently enter the pocket.

The zone 13 may be formed by compression of the absorbent core 4. The zone 13 may be formed by having a higher concentration of SAP on the surrounding parts of the zone 13 than on the zone area 13 in the absorbent core 4. The zone 13 may have a SAP concentration within the range of 0-40%, wherein the surrounding parts of the absorbent core 4 may have a SAP concentration within the range of 20-100%. Based on the above, the absorbent article 1 may comprise an unused state and a expanded state, wherein the thickness t1 of said zone 13 and the thickness t2 of the surrounding parts of the absorbent core 4 may be generally equal during the unused state of said absorbent article 1, wherein the thickness t2 of said surrounding parts of the absorbent core 4 may be generally greater than the thickness t1 of said zone when said absorbent core 4 is in a expanded state. In other words, the zone 13 may be constructed to have the same thickness t1 as the surrounding parts of the absorbent core 4 during its unused or dry state i.e. t1 = t2. During swelling of the absorbent core 4, the zone 13 will achieve a lower thickness compared to surrounding parts of the absorbent core i.e. t2 > t1. The unused state of the absorbent core 4 is the state where the absorbent article 1 is dry and no bodily exudates have been absorbed by the absorbent article 1. The expanded state is when the SAP in the absorbent core 4 have absorbed any bodily exudates.

The zone 13 may comprise a length I1 and the absorbent core 4 may comprise a length I2. The length I1 of the zone and the length I2 of the absorbent core 4 may be the same, in other words, said zone 13 may extend along the entire longitudinal length of the absorbent core 4, this is further disclosed in Figure 2A showing that the zone 13 extends along the whole longitudinal length of the absorbent core e.g. I1 = I2. Figure 2A further shows that the zone 13 is only partially recessed. The partial recess depicted in Figure 2A helps to form cushions on the side edges of the absorbent core 4 extending along the whole longitudinal length of the absorbent core 4. As seen in Figure 2A, the zone 13 may be in the form of a rectangle extending over the whole longitudinal length of the absorbent core 4.

The length-ratio between said zone 13 and said absorbent core 4 may be within the interval 0.04-1. The length-ratio is measured by dividing the length of the zone 13 with the length of the absorbent core 4. Further the zone 13 may comprise a width w1 and the absorbent core 4 may comprise a width w2, wherein the width-ratio between said zone 13 and said absorbent core 4 may be within the interval 0.02-0.8, preferably within 0.02-0.28. The width-ratio is measured by dividing the width of the zone 13 with the width of the absorbent core 4.

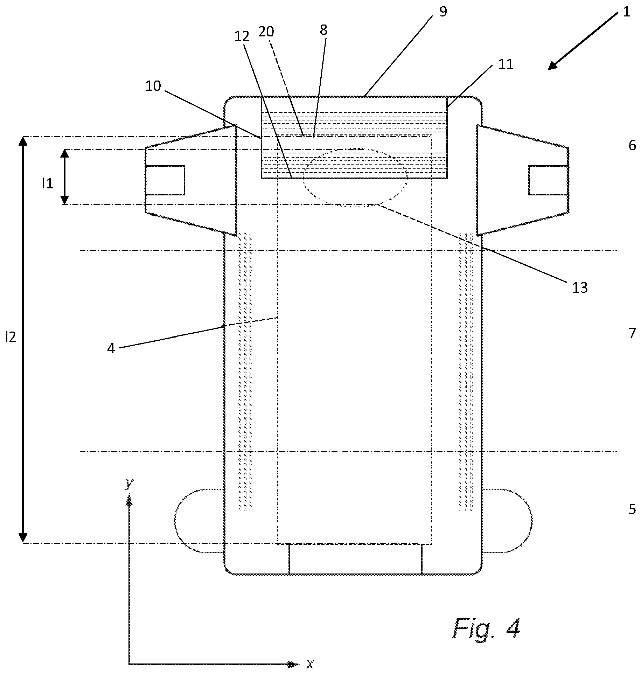

The absorbent article 1 of Figure 2A and Figure 2B shows that the zone 13 is partially recessed compared to surrounding parts of the absorbent core 4. Further, as shown in Figure 1A, the zone 13 may extend longitudinally from the back edge 15 of the absorbent article 1 but not along the whole length I2 of the absorbent core 4. Figure 1A shows that the zone 13 extends longitudinally from the back transversal edge 20 of the absorbent core 4 and has a u-shape. The zone 13 may further have the shape of a semi-circle, a V-shape or a rectangular shape extending towards said front edge 14 in the longitudinal direction. Further the zone 13 may have a circular or an oval shape, being centrally oriented on the absorbent core 4 in relation to the transversal axis x, however the circular or oval shape may be offset from the back transversal edge 20 of the absorbent core 4, in other words the zone 13 may be positioned to form a centrally oriented circular/oval hole in the absorbent core 4, or a centrally oriented circular/oval recess in the absorbent core 4, this is disclosed in Figure 4, where there is an at least partial recess being oval.

Further, the inboard transversal edge 12 of said capture layer 8 may at least partially overlap said zone 13. This is further shown in Figure 1A and Figure 2A. The benefit of having the inboard transversal edge 12 of the capture layer 8 to at least partially overlap the zone 13 is that the bodily exudates will efficiently enter the pocket formed by the capture layer 8 even if the absorbent core 4 is already expanded. Swelling of the core 4 may result in the pocket obtaining a greater volume, however, this would not block or hinder the function of the pocket in any way due to the capture layer 8 overlapping the zone 13.

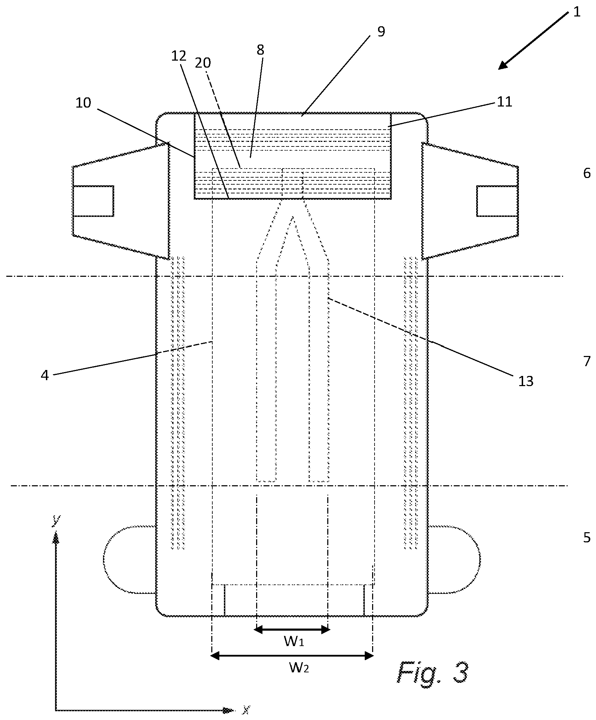

Figure 1B shows a cross-sectional front view of the absorbent article 1 in Figure 1A that depicts an absorbent article 1 with a fully recessed zone 13. As seen in Figure 1B, the absorbent core 4 is enclosed by a core cover 16 having a top-layer 17 and a bottom-layer 18, as Figure 1C shows, the top-layer 17 may be joined to the bottom-layer 18 in at least the area where the absorbent core 4 is fully recessed. Furthermore, the topsheet 2 may be at least partially joined to the top-layer 17 of the core cover 16 and the backsheet 3 may be at least partially joined to the bottom-layer 18 of the core cover 16. The joining may be by means of any method involving bonding, sealing or welding. For instance, adhesive bonding, glue sealing, ultrasonic bonding, ultrasonic sealing, thermal bonding or any other suitable method. The absorbent article 1 could have a tendency to be spanned over the area of the recess when the absorbent core 4 swells, resulting in that the zone 13 may not function properly, this may be avoided by joining the top-layer 17 to the bottom layer 18 in at least the area where the absorbent core 4 is fully recessed. The absorbent article 1 may have a zone 13 which is divided in said back portion 6 of the absorbent article 1 into two oblong channels extending longitudinally to at least said crotch portion 7 of said absorbent article 1 as disclosed in Figure 3. Figure 3 discloses that the zone 13 starts extending from the back transversal edge 20 of the absorbent core 4 and extends longitudinally to at least the crotch portion 7. The zone 13 in Figure 3 is divided into two channels in the back portion 6 of the absorbent article 1. The dividing of the zone 13 may be started right after the zone 13 overlaps the inboard transversal edge 12 of the capture layer 8 as shown in Figure 3, or the zone 13 may be divided before the zone 13 overlaps the inboard transversal edge 12 of the capture layer 8. The zone 13 may have a Y-shape as shown in Figure 3.

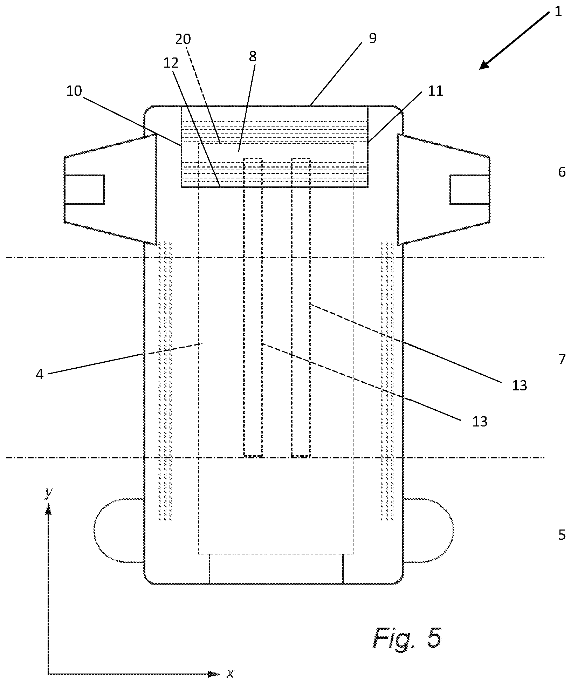

The absorbent article 1 may have two centrally oriented zones 13 extending from said back portion 6 to at least said crotch portion 7 as depicted in Figure 5. The zones 13 in Figure 5 are formed as two longitudinally extending rectangles that starts from the back portion 6 of the absorbent article 1 and overlaps the inboard transversal edge 12 of the capture layer 8 extending to at least the crotch portion 7 of the absorbent article 1. The benefit of the pattern of the zones 13 as shown in Figure 3 and Figure 5 is that the zone 13 may act as both a pressure relieving area helping fecal material to easier enter the pocket beneath the capture layer 8 and further to also help the core to be more easily formed around a user, optimizing comfort.

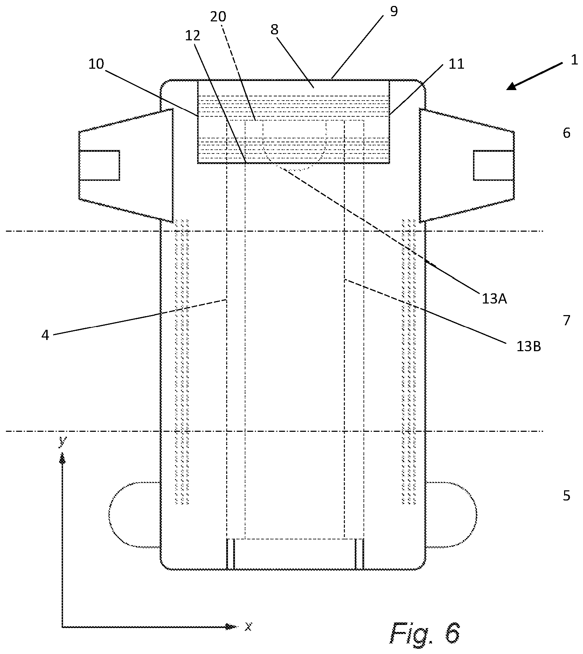

The absorbent articles 1 shown in Figure 1A and Figure 2A may be combined, in other words there may be an absorbent article 1 with a fully recessed centrally oriented zone 13 and further a partially recessed centrally oriented zone 13 within one absorbent article 1. This is disclosed in Figure 6, where the fully recessed centrally oriented zone 13A may then be positioned within the partially recessed centrally oriented zone 13B.

The invention may be an array of disposable pant-type absorbent articles 1 comprising two or more sizes. Absorbent articles 1 in the array may be manufactured and/or distributed by a single manufacturer, under a common brand name and/or under a common tradename or trademark. In certain embodiments, the array comprises absorbent articles 1 of different sizes. Additionally, or alternatively, the array may comprise absorbent articles 1 suited for different wearer and/or purchaser segments.

There is also provided a method for manufacturing an absorbent article 1 comprising a liquid-permeable topsheet 2, a liquid impermeable backsheet 3 and an absorbent core 4 positioned intermediate the liquid permeable topsheet 2 and the liquid impermeable backsheet 3, each layer having a skin-facing side and a garment-facing side, wherein said absorbent core 4 comprises a thickness t2, said absorbent article 1 being arranged along a longitudinal axis y and a transversal axis x extending in a perpendicular direction in relation to the longitudinal axis y, and said absorbent article 1 defining a front portion 5 having a front edge 14, a back portion 6 having a back edge 15 and a crotch portion 7 intermediate said front portion and said back portion, said method comprising:
- forming a capture layer 8 on said back portion of said absorbent article, wherein said capture layer 8 comprises an outboard transversal edge 9 facing said back edge 15 and two longitudinal edges 10, 11 being sealed to any part of said absorbent article 1, wherein said capture layer 8 further comprises an inboard transversal edge 12 facing said front edge 14, said inboard transversal edge being unsealed for gathering of feces, wherein said capture layer 8 is positioned to at least partially cover the skin-facing side of the topsheet 2

- forming an absorbent core 4 comprising at least one thickness, wherein wherein at least said back portion 6 of said absorbent core 4 further comprises at least one centrally oriented zone 13 in said absorbent core, said zone 13 comprising a thickness t1, wherein said zone 13 is at least partially recessed, resulting in said zone 13 having a generally lower thickness in relation to the thickness t2 of surrounding parts of the absorbent core 4.

The disclosure may be varied within the scope of the appended claims. For example, the absorbent article may further include leg elastics, prints, apertures, embossments, waist elastics etc as known to the skilled man in the art depending of the type of absorbent article intended.

## Claims

1. An absorbent article (1) comprising a liquid-permeable topsheet (2), a liquid impermeable backsheet (3) and an absorbent core (4) positioned intermediate the liquid permeable topsheet (2) and the liquid impermeable backsheet (3), each layer having a skin-facing side and a garment-facing side, wherein said absorbent core comprises a thickness (t2), said absorbent article (1) being arranged along a longitudinal axis (y) and a transversal axis (x) extending in a perpendicular direction in relation to the longitudinal axis (y), and said absorbent article (1) defining a front portion (5) having a front edge (14), a back portion (6) having a back edge (15) and a crotch portion (7) intermediate said front portion (5) and said back portion (6), wherein said back portion (6) of said absorbent article (1) further comprises a transversally extending capture layer (8) , wherein said capture layer (8) comprises an outboard transversal edge (9) facing said back edge (15) and two longitudinal edges (10, 11) being sealed to any part of said absorbent article (1), wherein said capture layer (8) further comprises an inboard transversal edge (12) facing said front edge (14), said inboard transversal edge (12) being unsealed for gathering of feces, wherein said capture layer (8) is positioned to at least partially cover the skin-facing side of the topsheet (2), wherein at least said back portion (6) of said absorbent article (1) further comprises at least one centrally oriented zone (13) in said absorbent core (4), said zone (13) comprising a thickness (t1), wherein said zone (13) is at least partially recessed, resulting in said zone (13) having a generally lower thickness in relation to the thickness (t2) of surrounding parts of the absorbent core (4), **characterized in that** said absorbent core (4) comprises a back transversal edge (20), wherein said at least one zone (13) extends longitudinally from said back transversal edge (20), wherein said inboard transversal edge (12) of said capture layer (8) at least partially overlaps said at least one zone (13).

2. The absorbent article (1) according to claim 1, wherein said at least one zone (13) have a lower basis weight in relation to surrounding parts of the absorbent core (4).

3. The absorbent article (1) according to any of the claims 1-2, wherein said at least one zone (13) comprises a length (I1), wherein said absorbent core (4) comprises a length (I2), wherein the length-ratio between said zone (13) and said absorbent core (4) is within the interval 0.04-1.

4. The absorbent article (1) according to any of the claims 1-3, wherein said at least one zone (13) comprises a width (w1), wherein said absorbent core (4) comprises a width (w2), wherein the width-ratio between said zone and said absorbent core (4) is within the interval 0.02-0.8.

5. The absorbent article (1) according to any of the claims 1-4, wherein said capture layer (8) comprises at least one transversally extending elastic strand positioned intermediate a pair of sealed layers of material.

6. The absorbent article (1) according to any of claims 1-5, wherein said at least one zone (13) is shaped generally as a semi-circle, a U-shape, a V-shape, a circular shape, an oval shape or a rectangular shape extending towards said front edge (14) in said longitudinal direction (y).

7. The absorbent article (1) according to any of claims 1-6, wherein the basis weight of said at least one zone (13) is within the interval of 0-200 g/m ², wherein the basis weight of surrounding parts of the absorbent core (4) is within the interval of 50-800 g/m ².

8. The absorbent article (1) according to any of claims 1-7, wherein the concentration of SAP is lower in the zone (13) compared to surrounding parts of the absorbent core (4).

9. The absorbent article (1) according to any of the claims 1-8, wherein said at least one zone (13) is fully recessed.

10. The absorbent article (1) according to claim 8, wherein said absorbent core (4) is enclosed by a core cover (16), comprising a top-layer (17) and a bottom-layer (18), wherein at least a part of said topsheet (2) or an intermediate layer is joined to said top-layer (17), wherein said top layer (17) and said bottom layer (18) are joined together in at least the area comprising said fully recessed zone (13).

11. The absorbent article (1) according to any of the claims 1-8, wherein said at least one zone (13) is partially recessed and extends along the entire longitudinal length of the absorbent core (4).

12. The absorbent article (1) according to any of the claims 1-10, wherein said at least one zone (13) is divided in said back portion (6) into two oblong channels extending longitudinally to at least said crotch portion (7) of said absorbent article (1).

13. The absorbent article (1) according to claim any of the claims 1-10, wherein said absorbent article (1) comprises two centrally oriented zones (13) extending from said back portion (6) to at least said crotch portion (7).

14. A method for manufacturing an absorbent article (1) comprising a liquid-permeable topsheet (2), a liquid impermeable backsheet (3) and an absorbent core (4) positioned intermediate the liquid permeable topsheet (2) and the liquid impermeable backsheet (3), each layer having a skin-facing side and a garment-facing side, wherein said absorbent core (4) comprises a thickness (t2), said absorbent article (1) being arranged along a longitudinal axis (y) and a transversal axis (x) extending in a perpendicular direction in relation to the longitudinal axis (y), and said absorbent article (1) defining a front portion (5) having a front edge (14), a back portion (6) having a back edge (15) and a crotch portion (7) intermediate said front portion and said back portion, said method comprising:
- forming a capture layer on said back portion of said absorbent article, wherein said capture layer (8) comprises an outboard transversal edge (9) facing said back edge and two longitudinal edges (10, 11) being sealed to any part of said absorbent article (1), wherein said capture layer (8) further comprises an inboard transversal edge (12) facing said front edge (14), said inboard transversal edge being unsealed for gathering of feces, wherein said capture layer (8) is positioned to at least partially cover the skin-facing side of the topsheet (2)
- forming an absorbent core (4) comprising at least one thickness (t), wherein wherein at least said back portion (6) of said absorbent core (4) further comprises at least one centrally oriented zone (13) in said absorbent core (4), said zone (13) comprising a thickness (t1), wherein said zone (13) is at least partially recessed, resulting in said zone (13) having a lower thickness (t1) in relation to the thickness (t2) of surrounding parts of the absorbent core (4), wherein said absorbent core (4) comprises a back transversal edge (20) wherein said at least one zone (13) extends longitudinally from said back transversal edge (20), wherein said inboard transversal edge (12) of said capture layer (8) at least partially overlaps said at least one zone (13).

## Patentansprüche

1. Saugfähiger Artikel (1), umfassend eine flüssigkeitsdurchlässige Deckfolie (2), eine flüssigkeitsundurchlässige Rückfolie (3) und einen saugfähigen Kern (4), der zwischen der flüssigkeitsdurchlässigen Deckfolie (2) und der flüssigkeitsundurchlässigen Rückfolie (3) positioniert ist, wobei jede Schicht eine der Haut zugewandte Seite und eine der Kleidung zugewandte Seite aufweist, wobei der saugfähige Kern eine Dicke (t2) umfasst, wobei der saugfähige Artikel (1) entlang einer Längsachse (y) und einer Querachse (x) angeordnet ist, die sich in einer senkrechten Richtung in Bezug auf die Längsachse (y) erstreckt, und der saugfähige Artikel (1) einen vorderen Abschnitt (5), der einen vorderen Rand (14) aufweist, einen hinteren Abschnitt (6), der einen hinteren Rand (15) aufweist, und einen Schrittabschnitt (7) zwischen dem vorderen Abschnitt (5) und dem hinteren Abschnitt (6) definiert, wobei der hintere Abschnitt (6) des saugfähigen Artikels (1) weiter eine sich quer erstreckende Auffangschicht (8) umfasst, wobei die Auffangschicht (8) einen äußeren, quer verlaufenden Rand (9), der dem hinteren Rand (15) zugewandt ist, und zwei längs verlaufende Ränder (10, 11) umfasst, die mit jedem Teil des saugfähigen Artikels (1) versiegelt sind, wobei die Auffangschicht (8) weiter einen inneren, quer verlaufenden Rand (12) umfasst, der dem vorderen Rand (14) zugewandt ist, wobei der innere, quer verlaufende Rand (12) zum Sammeln von Fäkalien unversiegelt ist, wobei die Auffangschicht (8) positioniert ist, um mindestens teilweise die der Haut zugewandte Seite der Deckfolie (2) zu bedecken, wobei mindestens der hintere Abschnitt (6) des saugfähigen Artikels (1) weiter mindestens eine zentral orientierte Zone (13) in dem saugfähigen Kern (4) umfasst, wobei die Zone (13) eine Dicke (t1) umfasst, wobei die Zone (13) mindestens teilweise ausgespart ist, was dazu führt, dass die Zone (13) eine allgemein geringere Dicke in Bezug auf die Dicke (t2) der umgebenden Teile des saugfähigen Kerns (4) aufweist, **dadurch gekennzeichnet, dass** der saugfähige Kern (4) einen hinteren, quer verlaufenden Rand (20) umfasst, wobei sich die mindestens eine Zone (13) in Längsrichtung von dem hinteren, quer verlaufenden Rand (20) erstreckt, wobei der innere, quer verlaufende Rand (12) der Auffangschicht (8) die mindestens eine Zone (13) mindestens teilweise überlappt.

2. Saugfähiger Artikel (1) nach Anspruch 1, wobei die mindestens eine Zone (13) ein geringeres Flächengewicht in Bezug auf die umgebenden Teile des saugfähigen Kerns (4) aufweist.

3. Saugfähiger Artikel (1) nach einem der Ansprüche 1-2, wobei die mindestens eine Zone (13) eine Länge (11) umfasst, wobei der saugfähige Kern (4) eine Länge (I2) umfasst, wobei das Längenverhältnis zwischen der Zone (13) und dem saugfähigen Kern (4) innerhalb des Intervalls 0,04-1 ist.

4. Saugfähiger Artikel (1) nach einem der Ansprüche 1-3, wobei die mindestens eine Zone (13) eine Breite (w1) umfasst, wobei der saugfähige Kern (4) eine Breite (w2) umfasst, wobei das Breitenverhältnis zwischen der Zone und dem saugfähigen Kern (4) innerhalb des Intervalls 0,02-0,8 ist.

5. Saugfähiger Artikel (1) nach einem der Ansprüche 1-4, wobei die Auffangschicht (8) mindestens einen sich quer erstreckenden elastischen Strang umfasst, der zwischen einem Paar versiegelter Materialschichten positioniert ist.

6. Saugfähiger Artikel (1) nach einem der Ansprüche 1-5, wobei die mindestens eine Zone (13) allgemein als ein Halbkreis, eine U-Form, eine V-Form, eine kreisförmige Gestalt, eine ovale Gestalt oder eine rechteckige Gestalt geformt ist, die sich in Richtung des vorderen Randes (14) in der Längsrichtung (y) erstreckt.

7. Saugfähiger Artikel (1) nach einem der Ansprüche 1-6, wobei das Flächengewicht der mindestens einen Zone (13) innerhalb des Intervalls von 0-200 g/m² ist, wobei das Flächengewicht der umgebenden Teile des saugfähigen Kerns (4) innerhalb des Intervalls von 50-800 g/m² ist.

8. Saugfähiger Artikel (1) nach einem der Ansprüche 1-7, wobei die Konzentration von SAP in der Zone (13) im Vergleich zu den umgebenden Teilen des saugfähigen Kerns (4) geringer ist.

9. Saugfähiger Artikel (1) nach einem der Ansprüche 1-8, wobei die mindestens eine Zone (13) vollständig ausgespart ist.

10. Saugfähiger Artikel (1) nach Anspruch 8, wobei der saugfähige Kern (4) von einer Kernabdeckung (16) umschlossen ist, die eine Oberschicht (17) und eine Unterschicht (18) umfasst, wobei mindestens ein Teil der Deckfolie (2) oder einer Zwischenschicht mit der Oberschicht (17) verbunden ist, wobei die Oberschicht (17) und die Unterschicht (18) mindestens in dem Bereich miteinander verbunden sind, der die vollständig ausgesparte Zone (13) umfasst.

11. Saugfähiger Artikel (1) nach einem der Ansprüche 1-8, wobei die mindestens eine Zone (13) teilweise ausgespart ist und sich über die gesamte Längslänge des saugfähigen Kerns (4) erstreckt.

12. Saugfähiger Artikel (1) nach einem der Ansprüche 1-10, wobei die mindestens eine Zone (13) in dem hinteren Abschnitt (6) in zwei längliche Kanäle unterteilt ist, die sich in Längsrichtung mindestens bis zum Schrittabschnitt (7) des saugfähigen Artikels (1) erstrecken.

13. Saugfähiger Artikel (1) nach einem der Ansprüche 1-10, wobei der saugfähige Artikel (1) zwei zentral orientierte Zonen (13) umfasst, die sich vom hinteren Abschnitt (6) mindestens bis zum Schrittabschnitt (7) erstrecken.

14. Verfahren zum Herstellen eines saugfähigen Artikels (1), umfassend eine flüssigkeitsdurchlässige Deckfolie (2), eine flüssigkeitsundurchlässige Rückfolie (3) und einen saugfähigen Kern (4), der zwischen der flüssigkeitsdurchlässigen Deckfolie (2) und der flüssigkeitsundurchlässigen Rückfolie (3) positioniert ist, wobei jede Schicht eine der Haut zugewandte Seite und eine der Kleidung zugewandte Seite aufweist, wobei der saugfähige Kern (4) eine Dicke (t2) umfasst, wobei der saugfähige Artikel (1) entlang einer Längsachse (y) und einer Querachse (x) angeordnet ist, die sich in einer senkrechten Richtung in Bezug auf die Längsachse (y) erstreckt, und wobei der saugfähige Artikel (1) einen vorderen Abschnitt (5), der einen vorderen Rand (14) aufweist, einen hinteren Abschnitt (6), der einen hinteren Rand (15) aufweist, und einen Schrittabschnitt (7) zwischen dem vorderen Abschnitt und dem hinteren Abschnitt definiert, wobei das Verfahren Folgendes umfasst:
- Ausbilden einer Auffangschicht auf dem hinteren Abschnitt des saugfähigen Artikels, wobei die Auffangschicht (8) einen äußeren, quer verlaufenden Rand (9) umfasst, der dem hinteren Rand zugewandt ist
und zwei längs verlaufende Ränder (10, 11), die mit irgendeinem Teil des saugfähigen Artikels (1) versiegelt sind, wobei die Auffangschicht (8) weiter einen inneren, quer verlaufenden Rand (12) umfasst, der dem vorderen Rand (14) zugewandt ist, wobei der innere, quer verlaufende Rand zum Sammeln von Fäkalien unversiegelt ist, wobei die Auffangschicht (8) positioniert ist, um mindestens teilweise die der Haut zugewandte Seite der Deckfolie (2) zu bedecken
- Ausbilden eines saugfähigen Kerns (4), der mindestens eine Dicke (t) umfasst, wobei mindestens der hintere Abschnitt (6) des saugfähigen Kerns (4) weiter mindestens eine zentral orientierte Zone (13) in dem saugfähigen Kern (4) umfasst, wobei die Zone (13) eine Dicke (t1) umfasst, wobei die Zone (13) mindestens teilweise ausgespart ist, was dazu führt, dass die Zone (13) eine geringere Dicke (t1) in Bezug auf die Dicke (t2) der umgebenden Teile des saugfähigen Kerns (4) aufweist, wobei der saugfähige Kern (4) einen hinteren, quer verlaufenden Rand (20) umfasst, wobei sich die mindestens eine Zone (13) in Längsrichtung von dem hinteren, quer verlaufenden Rand (20) erstreckt, wobei der innere, quer verlaufende Rand (12) der Auffangschicht (8) die mindestens eine Zone (13) mindestens teilweise überlappt.

## Revendications

1. Un article absorbant (1) comprenant une feuille supérieure perméable aux liquides (2), une feuille arrière imperméable aux liquides (3) et un noyau absorbant (4) positionné intermédiairement à la feuille supérieure perméable aux liquides (2) et la feuille arrière imperméable aux liquides (3), chaque feuille ayant un côté face à la peau et un côté face à l'habit, où ledit noyau absorbant comprend une épaisseur (t2), ledit article absorbant (1) étant disposé selon un axe longitudinal (y) et un axe transversal (x) s'étendant dans une direction perpendiculaire en relation à l'axe longitudinal (y), et ledit article absorbant (1) définissant une partie avant (5) ayant un bord avant (14), une partie arrière (6) avec un bord arrière (15) et une partie entrejambe (7) intermédiaire à ladite partie avant (5) et à ladite partie arrière (6), où ladite partie arrière (6) dudit article absorbant (1) comprend en outre une couche de capture (8) s'étendant transversalement, où ladite couche de capture (8) comprend un bord transversal extérieur (9) faisant face audit bord arrière (15) et deux bords longitudinaux (10, 11) scellés à n'importe quelle partie dudit article absorbant (1), où ladite couche de capture (8) comprend en outre un bord transversal intérieur (12) faisant face audit bord avant (14), ledit bord transversal intérieur (12) étant non scellé pour la collecte des excréments, où ladite couche de capture (8) est positionnée pour couvrir au moins partiellement le côté face à la peau de la feuille supérieure (2), où au moins la partie arrière (6) dudit article absorbant (1) comprend en outre au moins une zone orientée centralement (13) dans ledit noyau absorbant (4), ladite zone (13) comprenant une épaisseur (t1), où ladite zone (13) est au moins partiellement en retrait, ce dont il résulte que ladite zone (13) a une épaisseur généralement inférieure par rapport à l'épaisseur (t2) de parties environnantes du noyau absorbant (4), **caractérisé par le fait que** ledit noyau absorbant (4) comprend un bord transversal arrière (20), où ladite au moins une zone (13) s'étend longitudinalement à partir dudit bord transversal arrière (20), où ledit bord transversal intérieur (12) de ladite couche de capture (8) chevauche au moins partiellement ladite au moins une zone (13).

2. L'article absorbant (1) selon la revendication 1, dans lequel ladite au moins une zone (13) a un poids de base plus faible par rapport à des parties environnantes du noyau absorbant (4).

3. L'article absorbant (1) selon l'une des revendications 1 à 2, dans lequel ladite au moins une zone (13) comprend une longueur (11), où ledit noyau absorbant (4) comprend une longueur (I2), où le rapport de longueur entre ladite zone (13) et ledit noyau absorbant (4) se situe dans l'intervalle 0,04-1.

4. L'article absorbant (1) selon l'une des revendications 1 à 3, dans lequel ladite au moins une zone (13) comprend une largeur (w1), où ledit noyau absorbant (4) comprend une largeur (w2), où le rapport de largeur entre ladite zone et ledit noyau absorbant (4) se situe dans l'intervalle 0,02-0,8.

5. L'article absorbant (1) selon l'une des revendications 1 à 4, dans lequel ladite couche de capture (8) comprend au moins un brin élastique s'étendant transversalement positionné intermédiaire à une paire de couches de matériau scellées.

6. L'article absorbant (1) selon l'une des revendications 1 à 5, dans lequel ladite au moins une zone (13) est généralement formée en demi-cercle, en forme de U, en forme de V, en forme circulaire, en forme ovale ou en forme rectangulaire s'étendant vers ledit bord avant (14) dans la direction longitudinale (y).

7. L'article absorbant (1) selon l'une des revendications 1 à 6, dans lequel le poids de base de ladite au moins une zone (13) se situe dans l'intervalle de 0 à 200 g/m², où le poids de base de parties environnantes du noyau absorbant (4) se situe dans un intervalle de 50 à 800 g/m².

8. L'article absorbant (1) selon l'une des revendications 1 à 7, dans lequel la concentration de SAP est plus faible dans la zone (13) comparée à des parties environnantes du noyau absorbant (4).

9. L'article absorbant (1) selon l'une des revendications 1 à 8, dans lequel ladite au moins une zone (13) est entièrement en retrait.

10. L'article absorbant (1) selon la revendication 8, dans lequel ledit noyau absorbant (4) est enfermé par une couverture de noyau (16), comprenant une couche supérieure (17) et une couche inférieure (18), dans laquelle au moins une partie de ladite feuille supérieure (2) ou une couche intermédiaire est reliée à ladite couche supérieure (17), où ladite couche supérieure (17) et ladite couche inférieure (18) sont assemblées au moins dans la région comprenant ladite zone (13) entièrement en retrait.

11. L'article absorbant (1) selon n'importe quelle des revendications 1 à 8, dans lequel ladite au moins une zone (13) est partiellement en retrait et s'étend sur toute la longueur longitudinale du noyau absorbant (4).

12. L'article absorbant (1) selon l'une des revendications 1 à 10, dans lequel ladite au moins une zone (13) est divisé dans ladite partie arrière (6) en deux canaux oblongs s'étendant longitudinalement jusqu'à au moins ladite partie entrejambe (7) dudit article absorbant (1).

13. L'article absorbant (1) selon l'une des revendications 1 à 10, dans lequel ledit article absorbant (1) comprend deux zones orientées centralement (13) s'étendant depuis ladite partie arrière (6) jusqu'au moins ladite partie entrejambe (7).

14. Une méthode de fabrication d'un article absorbant (1) comprenant une feuille supérieure perméable aux liquides (2), une feuille arrière imperméable aux liquides (3) et un noyau absorbant (4) positionné intermédiaire à la feuille supérieure perméable aux liquides (2) et à la feuille arrière imperméable aux liquides (3), chaque feuille ayant un côté face à la peau et un côté face à l'habit, où ledit noyau absorbant (4) comprend une épaisseur (t2), ledit article absorbant (1) étant disposé selon un axe longitudinal (y) et un axe transversal (x) s'étendant dans un direction perpendiculaire par rapport à l'axe longitudinal (y), et ledit article absorbant (1) définissant une partie avant (5) ayant un bord avant (14), une partie arrière (6) ayant un bord arrière (15) et une partie entrejambe (7) intermédiaire à ladite partie avant et à ladite partie arrière, la méthode comprenant :
- former une couche de capture sur la partie arrière dudit article absorbant, où la couche de capture (8) comprend un bord transversal extérieur (9) faisant face audit bord arrière et deux bords longitudinaux (10, 11) scellés à n'importe quelle partie dudit article absorbant (1), où ladite couche de capture (8) comprend en outre un bord transversal intérieur (12) faisant face audit bord avant (14), ledit bord transversal intérieur étant déscellé pour la collecte des excréments, dans lequel la couche de capture (8) est positionnée pour couvrir au moins partiellement le côté face à la peau de la feuille supérieure (2),
- former un noyau absorbant (4) comprenant au moins une épaisseur (t), dans lequel dans lequel au moins ladite partie arrière (6) dudit noyau absorbant (4) comprend en outre au moins une zone orientée centralement (13) dans ledit noyau absorbant (4), ladite zone (13) comprenant une épaisseur (t1), où ladite zone (13) est au moins partiellement en retrait, ce dont il résulte que ladite zone (13) a une épaisseur (t1) plus faible par rapport à l'épaisseur (t2) de parties environnantes du noyau absorbant (4), dans lequel ledit noyau absorbant (4) comprend un bord transversal arrière (20) où ladite au moins une zone (13) s'étend longitudinalement à partir dudit bord transversal arrière (20), où le bord transversal intérieur (12) de ladite couche de capture (8) chevauche au moins partiellement ladite au moins une zone (13).
